Europäisches Patentamt

European Patent Office

Office européen des brevets

(11) Veröffentlichungsnummer: **0 357 944 B1**

(19)

# EUROPÄISCHE PATENTSCHRIFT

(12)

(12)

(45) Veröffentlichungstag der Patentschrift: **22.09.93**

(21) Anmeldenummer: **89114016.2**

(22) Anmeldetag: **28.07.89**

(51) Int. Cl.⁵: **A61B 6/14**, A61B 6/03, G01T 1/00

(54) Zahnärztliche Röntgendiagnostikeinrichtung zur Erstellung von Panorama-Schichtaufnahmen vom Kiefer eines Patienten.

(30) Priorität: **12.08.88 DE 3827474**

(43) Veröffentlichungstag der Anmeldung:
**14.03.90 Patentblatt 90/11**

(45) Bekanntmachung des Hinweises auf die Patenterteilung:
**22.09.93 Patentblatt 93/38**

(84) Benannte Vertragsstaaten:
**DE FR GB IT SE**

(56) Entgegenhaltungen:
**EP-A- 0 138 625**
**EP-A- 0 279 293**
**EP-A- 0 279 294**
**FR-A- 2 367 478**

(73) Patentinhaber: **SIEMENS AKTIENGESELL-SCHAFT**
**Wittelsbacherplatz 2**
**D-80312 München(DE)**

(72) Erfinder: **Schulze-Ganzlin, Ulrich, Dipl.-Ing-(TH)**
**Seehofstrasse 1**
**D-6143 Lorsch(DE)**
Erfinder: **Schwotzer, Axel, Dipl.-Ing. (TH)**
**Georgenstrasse 32**
**D-6087 Büttelborn(DE)**
Erfinder: **Pfeiffer, Joachim, Dr.**
**Eifelstrasse 33**
**D-6140 Bensheim(DE)**

## Beschreibung

Die Erfindung bezieht sich auf eine Röntgendiagnostikeinrichtung derjenigen Gattung, wie sie in der europäischen, lediglich zum Stand der Technik gemäß Artikel 54(3) und (4) EPÜ gehörenden Patentanmeldung EP-A-0279 294 beschrieben ist. Eine solche Einrichtung unterscheidet sich von konventionellen Panorama-Schichtaufnahmegeräten dadurch, daß anstelle des hinter der Sekundärblende vorbeibewegten Röntgenfilmes eine ortsfeste CCD-Sensoranordnung vorgesehen ist, welche mit Hilfe eines Taktgenerators so angesteuert wird, daß die erhaltenen Ladungsbilder mit der gleichen Geschwindigkeit in eine Speicherzone getaktet und dann zeilenweise über ein Shift-Register wieder ausgetaktet werden, mit der ein Röntgenfilm in der konventionellen Aufnahmetechnik relativ zum Sekundärspalt bewegt wird. Die Taktfrequenz ist dabei nach der Beziehung

$$f_{Takt} = \frac{v}{n_x \cdot a}$$

gewählt, wobei v die Filmgeschwindigkeit, $n_x$ das Abbildungsverhältnis des bildübertragenden Systems und a den Zeilenabstand darstellen.

Der im Anspruch 1 angegebenen Erfindung liegt die Aufgabe zugrunde, bei einem Röntgendiagnostikgerät der vorgenannten Gattung eine Möglichkeit anzugeben, während einer Aufnahme gleichzeitig mehrere Schichten erfassen zu können, wobei die Schichten innerhalb gewisser Grenzen frei bestimmbar sein sollen. Es soll ferner eine Möglichkeit gegeben sein, aussagekräftige Aufnahmen auch von Schichten, die nicht in einer Ebene liegen, vielmehr einen gekrümmten Verlauf einnehmen, z.B. von schräg im Kiefer liegenden Zähnen, erstellen zu können.

Dadurch, daß zumindest einige der CCD-Spalten der CCD-Sensoranordnung über getrennte Takteingänge mit unterschiedlicher Taktfrequenz angesteuert werden, lassen sich mehrere Schichten während eines Aufnahmeablaufs gleichzeitig erfassen. Jede Schicht kann dabei innerhalb gewisser Grenzen frei definiert werden.

Die unterschiedliche Ansteuerung braucht nicht gleichmäßig über den CCD-Sensor vorgenommen zu sein; sie kann vorteilhafterweise auf bestimmte Flächenbereiche des CCD-Sensors bzw. der Sensoranordnung konzentriert werden. Die Taktfrequenzen von Gruppen von Spalten können so aufgeteilt werden, daß die aufgenommene Schicht einen gekrümmten Verlauf einnimmt, so daß sich auch schräg im Kiefer liegende Zähne scharf abbilden lassen.

Die Tiefenschärfe der erstellten Schicht kann durch Veränderung der Bildzonenbreite des Sensors variiert werden, indem einige Zeilen des Sensors passiviert werden.

Weitere Vorteile ergeben sich aus der nachfolgenden Beschreibung eines Ausführungsbeispieles.
Es zeigen:
Figur 1 in einer Übersichtsdarstellung die wesentlichen Teile einer zahnärztlichen Röntgendiagnostikeinrichtung gemäß der Erfindung,
Figur 2 einen Teil der Strahlenaufnahmeeinheit in schaubildlicher Darstellung,
Figur 3 eine Variante zu der in Figur 2 dargestellten Ausführungsform.
Figur 4 ein Prinzipschaltbild zur Aufbereitung und Auswertung der Signale,
Figuren 5 und 6 eine vereinfachte Darstellung von Sekundärspalt einerseits und CCD-Sensor andererseits,
Figur 7 eine Prinzipdarstellung, betreffend die Ansteuerung der Spalten mit unterschiedlicher Frequenz,
Figuren 8 bis 10 eine Prinzipdarstellung des Vorganges einer Unterbrechung des Ladungstransports durch Ableiten von Ladungen.

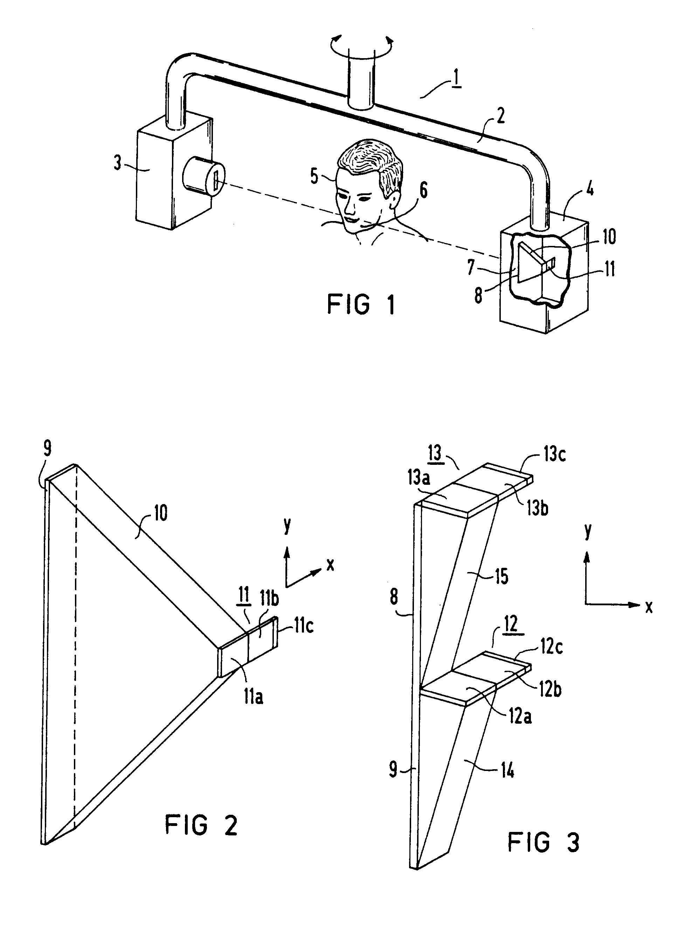

Die Figur 1 zeigt in stark vereinfachter perspektivischer Darstellung die wesentlichen Teile einer zahnärztlichen Röntgendiagnostikeinrichtung zur Erstellung von Panorama-Schichtaufnahmen. In bekannter Weise enthält die Diagnostikeinrichtung eine um eine lotrechte Achse schwenkbare Dreheinheit 1, bestehend aus einem Tragarm 2, an dessen einem Ende ein Röntgenstrahler 3 und an dessen anderem Ende eine Strahlenaufnahmeeinheit 4 gehaltert sind. Die Dreheinheit 1 ist in bekannter Weise relativ zum Patientenkopf 5 derart verstellbar, daß sich eine Übersichtsaufnahme vom gesamten Kiefer 6 eines Patienten erstellen läßt. Die hierzu erforderlichen Verstelleinrichtungen und Bewegungsabläufe, die die Dreheinheit ausführt, sind hinlänglich bekannt, brauchen deshalb nicht mehr im einzelnen erläutert zu werden.

Die Strahlenaufnahmeeinheit 4 enthält in bekannter Weise eine Blende 7 mit einem Sekundärspalt 8 in den Abmessungen von beispielsweise 5 x 125 mm. An den Sekundärspalt 8 schließen sich Mittel zur Konversion der Röntgenstrahlen in sichtbare Strahlen an. Im vorliegenden Ausführungsbeispiel ist hierzu (Figur 2) in Sekundärspaltebene eine Szintillatorschicht 9 in den Abmessungen des Sekundärspaltes vorgesehen. An die Szintillatorschicht 9 schließt sich ein Bildübertragungselement 10 in Form einer Faseroptik an, die das Format des Sekundärspaltes von 5 x 125 mm auf das Format der Bildzone eines CCD-Sensors 11 mit den Abmessungen von beispielsweise 8 x 8 mm verkleinert.

Bei dem CCD-Sensor 11 handelt es sich um einen Typ, bei dem Speicher- und Bildzone (11a, 11b) innerhalb des chips räumlich getrennt angeordnet sind und bei dem das shift-Register 11c mit der CCD-Speicherzone 11b gekoppelt ist. Anstelle dieses CCD-Typs kann auch ein CCD-Typ verwendet werden, bei dem Bild und Speicherzone zusammenliegend innerhalb eines chips angeordnet sind. In Verbindung mit der oben beschriebenen Analogie zwischen der Ladungsbewegung und der Filmbewegung ist es vorteilhaft, einen CCD-Sensor zu verwenden, der keine Speicherzone mehr enthält, bei dem also die Signale zeilenweise direkt in das shift-Register geschoben und dann ausgetaktet werden.

Um die Funktionsbeschreibung zu vereinfachen, wird in der nachfolgenden Beschreibung davon ausgegangen, daß der gesamte Sekundärspalt 8 auf der Oberfläche eines einzigen CCD-Sensors abgebildet wird, wie dies in Figur 2 dargestellt ist. Aus heutiger Sicht ist es jedoch angezeigt, mehrere CCD-Sensoren vorzusehen, um den Sekundärspalt 8 abzudecken, wie dies in Figur 3 dargestellt ist. Entsprechend dieser Darstellung sind zur Abbildung des Sekundärspaltes 8 zwei etwa rechtwinkelig zur Sekundärspaltebene angeordnete CCD-Sensoren 12 und 13 und, als Übertragungselemente, zwei faseroptische Elemente 14, 15 vorgesehen. Die einzelnen CCD-Sensoren sind gleichen Typs, wie in Figur 2, also mit von der Bildzone räumlich getrennter Speicherzone und anschließendem shift-Register. Wie oben erwähnt, können diese vorteilhafterweise auch ohne Speicherzone ausgebildet sein.

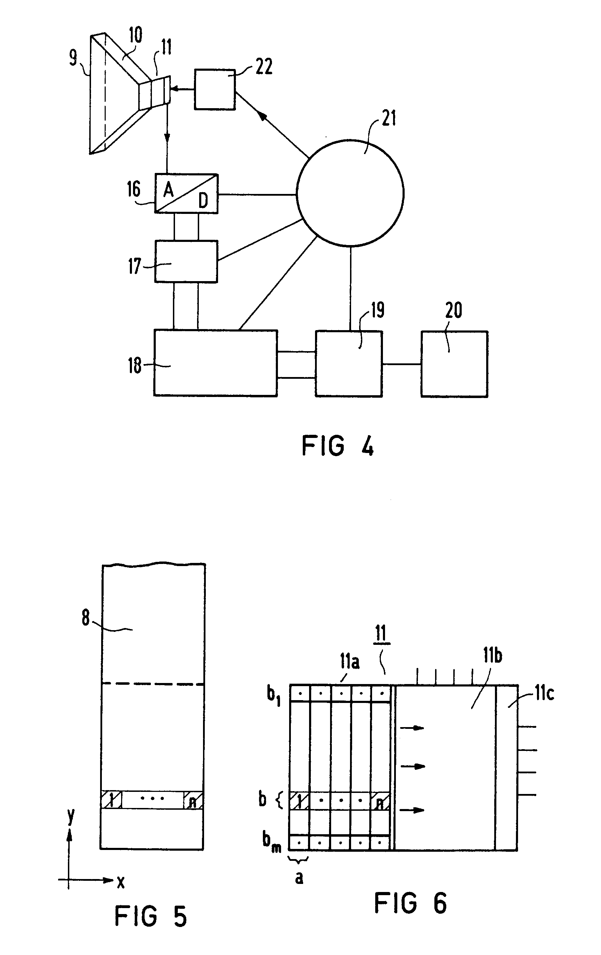

Die Signalverarbeitung wird anhand des Prinzipschaltbildes nach Figur 4 erläutert. Danach werden die aus der Detektoranordnung (CCD-Sensor/Sensoren) gewonnenen Signale nach Signalaufbereitung an einen A/D-Wandler 16 gegeben, an den sich ein digitales Bildverarbeitungssystem mit einer Vorverarbeitungseinheit 17, einem Bildspeicher 18, einer Bildausleseeinheit 19, einem Monitor 20 und einem Rechner 21 anschließen. Mit 22 ist schließlich ein später noch näher erläuterter Taktgenerator bezeichnet, mit dem die CCD-Sensoren angesteuert werden. Vorteilhafterweise können auch entsprechend den K-Gruppen von Spalten K-shift-Register, K-Signalaufbereitungen und K-A/D-Wandler verwendet werden.

Die am Ausgang eines shift-Registers 11c des CCD-Sensors 11 (Fig. 2) anliegenden Bildinformationen werden im zugehorigen A/D-Wandler digitalisiert und sodann entweder direkt in den Bildspeicher 18 eingegeben oder nach Vorverarbeitung in der Einheit 17 im Bildspeicher 18 abgespeichert. Der Rechner 21 gibt hierzu die notwendigen Steuer- bzw. Auslesebefehle.

Die direkte Eingabe der vom A/D-Wandler erhaltenen Signale in den Bildspeicher 18 ist dann vorteilhaft, wenn letzterer bei vertretbarem Kostenaufwand bzw. Bauvolumen eine entsprechend große Kapazität aufweist. In diesem Falle werden die Daten während eines Umlaufs der Dreheinheit zunächst nur abgespeichert und erst nach Abschluß der Aufnahme, also nach Umlauf des Strahlers um den Patientenkopf, verarbeitet, d.h. in der einer Filmaufnahme entsprechenden Geschwindigkeit aufaddiert. Wenngleich hierzu eine relativ große Datenmenge verarbeitet werden muß, so hat diese Lösung den Vorteil, daß eine Darstellung mehrerer beliebiger Schichten möglich ist.

Ist die unter vorgenanntem Gesichtspunkt notwendige Bildspeicherkapazität nicht mit wirtschaftlich vertretbarem Aufwand zu erhalten, so kann es vorteilhaft sein, die besagte Vorverarbeitungseinheit 17 zwischenzuschalten. Diese Vorverarbeitungseinheit enthält einen Zwischenspeicher und einen Arithmetikprozessor, wodurch die digitalen Daten aus dem A/D-Wandler entsprechend einem Steuerbefehl aus dem Rechner 21 zeitgerecht addiert, d.h. so zusammengesetzt werden, daß ein Schichtbild einer gewünschten Schichtlage entsteht. Die hieraus resultierenden, aufgearbeiteten Daten werden anschließend in den Bildspeicher 18 gegeben. Durch die Zwischenschaltung der Vorverarbeitungseinheit 17 kann also ein Bildspeicher mit kleinerer Kapazität verwendet werden. Allerdings ist die Schichtlage durch Zwischenschalten einer solchen Vorverarbeitungseinheit festgelegt, d.h. die Schichtlage kann nicht mehr in gewissen Grenzen verändert werden, im Gegensatz zu der vorerwähnten Alternativanordnung ohne Vorverarbeitungseinheit, wo die Bilddaten erst nach einer Aufnahme zu einem Schichtbild zusammengesetzt werden.

Zwischen beiden Möglichkeiten kann auch ein Kompromiß vorgesehen sein, gemäß dem in der Vorverarbeitungseinheit 17 vor der Abspeicherung in den Bildspeicher mehrere benachbarte Bildspalten zeitgerecht aufaddiert werden und die Addition dieser Summenspalten zu einer Bildspalte nach Abspeicherung erfolgt.

Nachstehend sei die Bildentstehung kurz erläutert:

Der Patient wird von einem durch eine im Röntgenstrahler 3 befindliche Primärblende begrenzten rechteckigen spaltförmigen Strahlenbündel durchstrahlt. Der Strahl trifft auf den Sekundärspalt 8 der Sekundärblende 7 und trifft sodann auf die Szintillatorschicht 9, wird dort in sichtbare Strahlen umgewandelt, die schließlich vom CCD-Sensor aufgenommen werden. Die vom CCD-Sensor aufgenommenen Signale sind proportional der Strahlungsintensität der dem Patientenkopf durchdringenden Röntgenstrahlung.

Bei konventioneller Schichtaufnahmetechnik wird hinter der Sekundärblende, also hinter dem Sekundärspalt 8, ein zu belichtender Röntgenfilm mit einer bestimmten Geschwindigkeit vorbeigezogen, wobei die gewählte Geschwindigkeit die Lage der tomographischen Schicht beeinflußt, d.h. durch Änderung der Filmgeschwindigkeit kann die tomographische Schicht geändert werden. Im vorliegenden Falle, wo der Röntgenfilm durch eine elektronische Detektoranordnung ersetzt wird, werden die am Detektor entstehenden Signale, wie nachfolgend näher erläutert, in bestimmter Weise zu einem auf einem Monitor wiedergebbaren Panorama-Schichtbild verarbeitet.

Anhand der Figuren 5 und 6 wird zunächst die Beziehung zwischen Sekundärspalt und der Bildzone des CCD-Sensors erläutert. Es wird davon ausgegangen, daß der Sekundärspalt 8 auf der Bildzone eines oder mehrerer CCD-Sensoren abgebildet wird. Das Abbildungsverhältnis in x-Richtung, also senkrecht zur Längsausdehnung des Sekundärspaltes 8, beträgt dabei 1 : $n_x$ und in y-Richtung 1 : $n_y$. Nachdem der Sekundärspalt eine Breite von etwa 5 mm und die heute verwendbaren CCD-Sensoren eine Bildzonenbreite von 8 mm haben, beträgt im Betrachtungsfalle $n_x = 1$. Je nach Anzahl und Größe der verwendeten Bildsensoren kann der Abildungsmaßstab $n_y$ in Spaltlängsrichtung zwischen 1 und etwa 20 liegen.

Ein Bildpunkt (Pixel) auf der CCD-Bildzonenfläche mit den Abmessungen a · b (a = Zeilenabstand, b = Spaltenabstand) entspricht einem Pixel von $(n_x a) · (n_y b)$ in der Sekundärspaltebene. In der vereinfachten Übersicht nach Figuren 4 und 5 entsprechen also 1 bis n Licht-Bildpunkte im Sekundärspalt 1 bis n Ladungs-Bildpunkten im CCD-Sensor. In Richtung der Längsausdehnung des Sekundärspaltes gilt Analoges. Demgemäß wird eine Linie in Richtung des Sekundärspaltes auf einer CCD-Zeile abgebildet.

Durch Anlegen entsprechender Taktimpulse über den Taktgenerator 22 wird das Bild aus der Bildzone 11a in die Speicherzone 11b transportiert und von dort über das shift-Register 11c ausgelesen und in den A/D-Wandler eingegeben. Bei Normalbetrieb, d.h. in der Standardtaktfolge eines CCD-Sensors beträgt die Bildintegrationszeit etwa 20 ms. Danach wird das Bild in die Speicherzone getaktet. Hierzu sind so viele Takte notwendig, wie der CCD-Sensor Zeilen hat. Unter Zugrundelegung eines CCD-Typs mit 300 Zeilen und eines Taktes von 2 $\mu$s ist die Bildzone also nach ca. 0,6 ms entleert und kann dann sofort ein neues Bild aufnehmen. Während bei konventioneller Schichtaufnahmetechnik der Film mit einer bestimmten Geschwindigkeit hinter dem Sekundärspalt vorbeibewegt wird, also während der Bewegung des Filmes die am Sekundärspalt befindliche Bildinformation über einen bestimmten Zeitraum auf dem Bild integriert wird, wird gemäß der Erfindung diese Integration der Bildinformation elektronisch imitiert, indem das am Szintillator entstehende Lichtbild auf der Oberfläche des CCD-Sensors abgebildet und das entstehende Ladungsbild in einer bestimmten Takt folge aus der Bildzone in die Speicherzone getaktet und dann zeilenweise über das shift-Register ausgetaktet wird. Die Taktfolge ist dabei so gewählt, daß das Ladungsbild, bezogen auf die Sekundärspaltebene, die gleiche Geschwindigkeit in x-Richtung hat, die ein Film bei konventioneller Schichtaufnahmetechnik haben würde. Die ermittelte Taktfrequenz steht in folgender Beziehung zu der Äquivalent-Geschwindigkeit (v) eines Filmes:

$$f_{Takt} = \frac{v}{n_x \cdot a}$$

wobei $f_{Takt}$ die Anzahl der Zeilen pro Sekunde und $(n_x \cdot a)$ den CCD-Zeilenabstand, bezogen auf die Sekundärspaltebene, angibt. Bei einer typischen Filmgeschwindigkeit von 30 mm/s und einem Zeilenabstand von 20 $\mu$m und unter Zugrundelegung eines Abbildungsverhältnisses von 1:1 in x-Richtung würde sich eine Taktfrequenz von 1.500 Hz ergeben.

Der Vorgang der Bildintegration sei hier nicht weiter erläutert. Hierzu wird auf die eingangs erwähnte europäische Patentanmeldung EP-A- 0279 294 verwiesen, in der die Diagrammfolgen zur Erläuterung der Bildintegration aufgezeigt sind.

Wie bereits angesprochen, ist das Vorhandensein einer Speicherzone beim CCD-Sensor nicht zwingend notwendig; es kann vielmehr vorteilhaft sein, die Ladungen aus der Bildzone zeilenweise direkt in das shift-Register zu schieben und dort auszutakten. Damit läßt sich ein unnötiger Ladungstransport vermeiden.

In Betrachtung der Figur 6 sei angenommen, daß der CCD-Sensor 11 in Spalten $b_1$ bis $b_m$ unterteilt ist. Wie bereits erläutert, wird durch Integration und Bewegen der Ladungen in der Bildzone 11a ein Ladungsbild erzeugt, das einem belichteten Bereich auf einem konventionell bestrahlten Röntgenfilm entspricht. Die Ladungen werden spaltenweise von einer CCD-Zeile zur nächsten verschoben und in die Speicherzone 11b oder direkt in ein Schieberegister 1c ausgetaktet. Die Geschwindigkeit, mit der dieser Vorgang erfolgt, wird von der Schiebe-Taktfrequenz vorgegeben. Eine scharf abgebildete Schicht ergibt sich aus folgender Beziehung:

$$f = \frac{v}{a} \cdot \frac{d}{(1-d)}$$

Dabei bedeuten:

$d$ = Abstand Film zu Objekt

$l$ = Abstand Film zu Strahlenquelle

$v$ = Geschwindigkeit Strahlenquelle senkrecht zum Objekt

$a$ = CCD-Zeilenbreite

$f$ = Taktfrequenz

Das Produkt $a \cdot f$ entspricht der Filmgeschwindigkeit bei konventioneller Röntgenaufnahmetechnik.

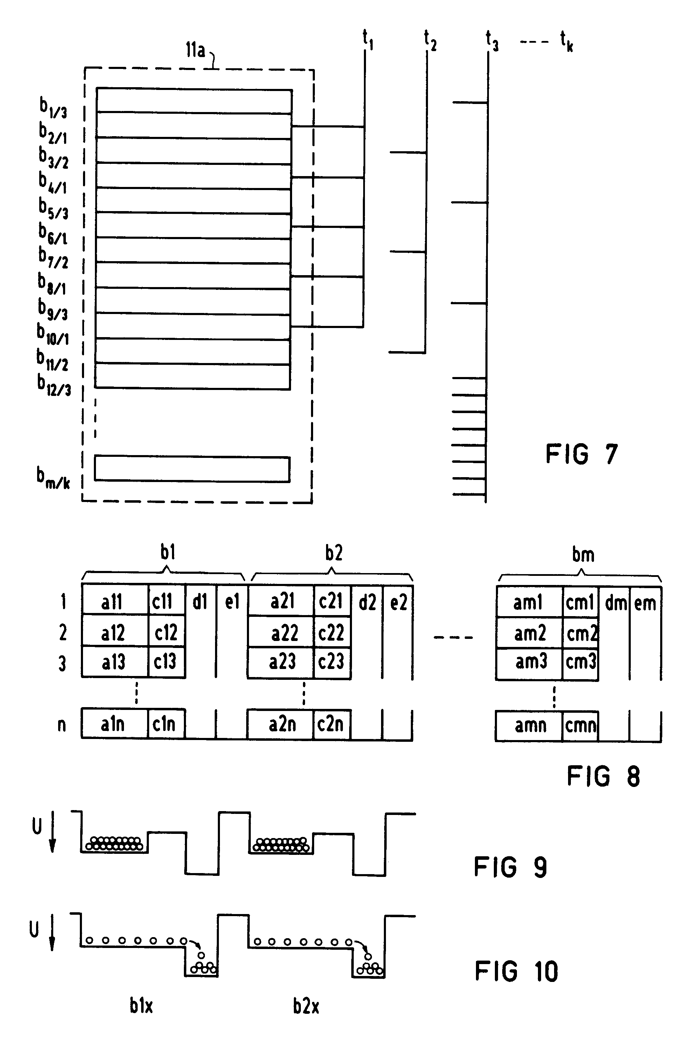

Wie anhand der Figur 7 aufgezeigt, werden einige der CCD-Spalten $b_1$ bis $b_m$ mit unterschiedlichen Taktfrequenzen $t_1$ bis $t_k$ angesteuert. Jede Taktfrequenz entspricht dabei einer Aufnahmeschicht. Die Ansteuerung kann gleichmäßig verteilt über den CCD-Sensor erfolgen; vorteilhaft ist es jedoch, die unterschiedliche Ansteuerung der Spalten auf bestimmte Flächenbereiche des CCD-Sensorelements, bzw. dann, wenn mehrere CCD-Sensorelemente einen Sekundärspalt abdecken, auf bestimmte Bereiche des Sekundärspaltes zu konzentrieren. Eine solche Konzentration auf bestimmte Flächenbereiche ist z.B. dann angebracht, wenn in manchen Bildbereichen mehrere Schichten erfaßt werden sollen und dabei eine Reduzierung der Bildauflösung in Kauf genommen werden kann, während in anderen Bildbereichen eine einzige Schicht genügt. Die Figur 7 zeigt eine solche Konzentration auf dem oberen Flächenabschnitt des CCD-Sensors. Im Ausführungsbeispiel werden mit der Taktfrequenz $t_1$ die Spalten $b_2$, $b_4$, $b_6$, $b_8$ und $b_{10}$, mit der Taktfrequenz $t_2$ die Spalten $b_3$, $b_7$ und $b_{11}$ und mit der Taktfrequenz $t_3$ die Spalten $b_1$, $b_5$, $b_9$ und $b_{12}$ und danach jede weitere Spalte angesteuert. Dies bedeutet, daß im oberen Abschnitt des CCD-Elements die Spalten mit drei verschiedenen, unterschiedlichen Schichten entsprechenden Taktfrequenzen angesteuert werden, während ab der zwölften Spalte der Sensor nur mit einer Taktfrequenz angesteuert wird. In der dargestellten Ausführung lassen sich also im oberen Bildabschnitt drei verschiedene Schichten entsprechend den Taktfrequenzen $t_1$, $t_2$ und $t_3$ erfassen, während im unteren Bildabschnitt lediglich eine Schicht, nämlich die Schicht entsprechend der Taktfrequenz $t_3$ abgebildet wird.

Vorteilhafterweise werden die mit gleicher Taktfrequenz angesteuerten Spalten zu Gruppen zusammengefaßt angesteuert; denkbar ist jedoch auch eine Einzelansteuerung. Zur Ansteuerung besitzt jede Spalte einen entsprechenden Takteingang, der, entsprechend Figur 4, mit dem Taktgenerator 22 verbunden ist.

Die Taktfrequenzen $t_1$ bis $t_k$ können vorteilhafterweise so aufgeteilt werden, daß vom Kiefer keine ebene, sondern eine gekrümmte Schicht aufgenommen wird.

Mit Hilfe des Rechners 21 und der Bildverarbeitungseinheit 17 lassen sich alle gewünschten Schichten unabhängig voneinander darstellen, weitere Zwischenschichten berechnen und auch mehrere Schichten zu einer Schicht mit größerem Tiefenschärfenbereich zusammenfassen.

Davon ausgehend, daß eine Veränderung des Tiefenschärfenbereiches durch eine Änderung der Breite der aktiven Sensorfläche erzielbar ist, wird gemäß einer Weiterbildung der Erfindung vorgeschlagen, durch wahlweises Passivieren von am Rand der Bildzone liegenden CCD-Zeilen die Breite des Spaltes während der Aufnahme "künstlich" zu beeinflussen. Das Passivieren bzw. Abtrennen von Zeilen läßt sich durch Ableiten von Ladungen in das Substrat (Masse) des CCD-Sensors erreichen. Ein solches elektrisches Abkoppeln der Ladungen kann vorteilhafterweise dadurch geschehen, daß auf dem CCD-Sensor integrierte Analog-Schalter vorgesehen sind, die von außen über entsprechend vorgesehene Signalleitungen aktiviert werden. Durch entsprechendes Aktivieren kann die aktive Sensorfläche beeinflußt werden, wobei einer größeren aktiven Sensorfläche ein kleinerer Tiefenschärfenbereich entspricht.

Anhand der Figuren 8 bis 10 wird eine Möglichkeit des Aus- bzw. Abkoppelns von Ladungen beschrieben, wobei als CCD-Sensor einer mit getrennter Bild- und Speicherzone oder ohne Speicherzone verwendet werden kann. Die Figur 8 zeigt zunächst in einer Prinzipdarstellung die Anordnung der fotoempfindlichen Zellen und Elektroden eines CCD-Sensors.

Es wird davon ausgegangen, daß jedes Pixel eines CCD-Sensors aus vier Bereichen besteht

- den fotoempfindlichen Zellen $a_{11}$, $a_{12}$, $a_{13}$ ... $a_{1n}$, welche in der Darstellung für n-Zeilen in jeder Spalte $b_1$, $b_2$ ... $b_m$ untereinander gezeichnet sind
- den Sperrelektroden $c_{11}$, $c_{12}$, $c_{13}$ ... $c_{1n}$ für jede Zelle mit (nicht dargestellten) Anschlüssen zum Steuern des Abkoppelvorganges
- den Potentialgräben $d_1$, $d_2$ ... $d_m$, in die die Ladungen nach dem Abkoppeln "abgesaugt" und zur Masse (Substrat des CCD-Sensors) geleitet werden und
- den Trennzonen $e_1$, $e_2$ ... $e_n$ zwischen den einzelnen Spalten $b_1$, $b_2$ ... $b_m$.

Wie die Figuren 9 und 10 zeigen, liegen die vier Bereiche auf vier verschiedenen Spannungspotentialen (U), wobei, wie durch den Pfeil angegeben, die Pfeilspitze auf ein höheres Spannungspotential deutet.

Normalerweise fließen die Ladungen zeilenweise (Zeilen 1, 2 ... n) von $a_{11}$, $a_{12}$ ... $a_{1n}$ bis zur Bildspeicherzone bzw., wenn eine solche nicht vorhanden ist, direkt bis zum Schieberegister. Die Verteilung der Spannungspotentiale für diese vier Bereiche ist in diesem Zustand in Figur 9 dargestellt.

Wird an einer bestimmten Stelle, z.B. bei $c_{13}$, die Sperrelektrode aktiviert, d.h. wird über einen entsprechenden Anschluß dort ein Signal gesetzt, so fallen sämtliche Ladungen von $a_{11}$ bis $a_{13}$ in den Potentialgraben $d_1$, wodurch diese Ladungen gelöscht werden. Dieser Zustand ist vereinfacht in Figur 10 dargestellt.

Durch gezieltes Ansteuern bestimmter Sperrelektroden kann der Ladungstransport an einer bestimmten Stelle der Bildebene unterbrochen und dadurch, wie oben bereits erwähnt, der Tiefenschärfenbereich variiert werden.

## Patentansprüche

1. Zahnärztliche Röntgendiagnostikeinrichtung zur Erstellung von Panorama-Schichtaufnahmen vom Kiefer eines Patienten, enthaltend folgende Merkmale:

    a) eine um eine vertikale Achse drehbare Einheit (1), die Träger einerseits einer Röntgenstrahlenquelle (3) und andererseits einer Blende (7) mit Sekundärspalt ist,

    b) Mittel (9) zur Konversion der Röntgenstrahlen in sichtbare Strahlen,

    c) eine Detektoranordnung (11) zur Bildung elektrischer Signale proportional zur Strahlungsintensität,

    d) einen mit der Detektoranordnung verbundenen A/D-Wandler (16), der die von der Detektoranordnung gewonnenen Spannungen digitalisiert,

    e) einen Bildspeicher (18),

    f) eine Datenverarbeitungseinrichtung mit Rechner (21), welche aus den von der Detektoranordnung während eines Aufnahmeablaufs gelieferten Signalen ein Übersichtsbild berechnet,

    g) eine an die Datenverarbeitungseinrichtung angeschlossene Bildwiedergabeeinrichtung (20),

    h) die Detektoranordnung enthält ein oder mehrere CCD-Sensoren (11), die so angeordnet sind, daß sich auf deren Bildzone (11a) der Sekundärspalt (8) abbilden läßt, wobei eine Linie in Richtung der Längsausdehnung des Spaltes auf einer CCD-Zeile abgebildet wird, i) mit dem CCD-Sensor (11) ist ein Taktgenerator (22) verbunden, durch dessen Taktimpulse Lichtbilder (LiB) im Sekundärspalt (8) entsprechende Ladungsbilder (LB) zu einem Gesamtladungsbild (LBges.) aufaddiert werden, wobei die Ladungsbilder (LB) aus der Bildzone (11a) gegebenenfalls in eine Speicherzone (11b) transportiert und danach über ein shiftRegister (11c) ausgelesen und anschließend in den A/D-Wandler (16) gegeben werden,

    j) die Taktfrequenz ($f_{Takt}$) des Taktgenerators (22) ist so gewählt, daß die Ladungsbilder (LB) mit der gleichen Geschwindigkeit (v) zeilenweise über das shift-Register (11c) ausgetaktet werden, mit der ein Röntgenfilm bei konventioneller Aufnahmetechnik relativ zum Sekundärspalt (8) bewegt wird.

    k) zumindest einige der CCD-Spalten ($b_1$ bis $b_m$) werden einzeln oder zu Gruppen ($b_{2/1}$, $b_{4/1}$ ... $b_{10/1}$; $b_{3/2}$, $b_{7/2}$, $b_{11/2}$) zusammengefaßt über getrennte Takteingänge mit unterschiedlicher Taktfrequenz ($t_1$, $t_2$, $t_3$ ... $t_k$) angesteuert.

2. Röntgendiagnostikeinrichtung nach Anspruch 1, **dadurch gekennzeichnet,** daß die unterschiedliche Ansteuerung der CCD-Spalten ($b_1$ ... $b_m$) auf bestimmte Flächenbereiche des CCD-Sensors (11) bzw. der Sensoranordnung (12, 13) konzentriert ist.

3. Röntgendiagnostikeinrichtung nach Anspruch 1 oder 2, **dadurch gekennzeichnet,** daß die Taktfrequenzen ($t_1$ bis $t_k$) mit Hilfe eines Taktgenerators (22) so aufteilbar sind, daß vom Kiefer eine gekrümmte Schicht aufgenommen wird.

4. Röntgendiagnostikeinrichtung nach einem der Ansprüche 1 bis 3, **dadurch gekennzeichnet,** daß durch elektrisches Abkoppeln von Ladungen am Rand der Bildzone (11a) des CCD-Sensors (11) die Breite der aktiven Sensorfläche (8) und damit die Tiefenschärfe der erstellten Schicht veränderbar ist.

5. Röntgendiagnostikeinrichtung nach Anspruch 4, **dadurch gekennzeichnet,** daß das Abkoppeln der Ladungen durch Unterbrechung des Ladungstransportes erfolgt, zu welchem Zweck der CCD-Sensor (11) integrierte Analogschalter enthält, die bestimmte Ladungen zum Substrat des CCD-Sensors bzw. zur Masse ableiten.

## Claims

1. A dental X-ray diagnostics device for preparing panorama-layer exposures of the jaw of a patient, containing the following features:

    a) a unit (1) able to be rotated about a vertical axis, which is the carrier, on the one hand, of an X-ray source (3), and on the other hand, a diaphragm (7) with a secondary gap,

b) means (9) for converting the X-rays into visible rays,

c) a detector arrangement (11) for forming electric signals proportional to the radiation intensity,

d) an A/D converter (16) connected to the detector arrangement, which digitises the voltages produced by the detector arrangement,

e) an image memory (18),

f) a data processing device with a computer (21), which calculates an overview display from the signals delivered by the detector arrangement during a recording operation,

g) an image reproduction device (20), connected to the data processing device,

h) the detector arrangement contains one or several CCD-sensors (11), which are arranged so that the secondary gap (8) can be displayed on the image zone (11a), wherein a line in the direction of the longitudinal extent of the gap is displayed on a CCD-row.

i) a clock generator (22) is connected to the CCD-sensor (11) through the clock pulses of which photographic images (LiB) in the secondary gap (8) are added to corresponding charge images (LB) to form a complete charge image (LBges), wherein the charge images (LB) are transferred from the image zone (11a), and if the case arises, into a storage zone (11b) and then read out by way of a shift register (11c) and subsequently transmitted to the A/D-converter (16),

j) a clock frequency ($f_{Takt}$) of the clock generator (22) is selected so that the charge images (LB) are clocked out in lines by way of the shift register (11c) with the same speed (v), with which an X-ray film is moved relative to the secondary gap (8) with conventional recording techniques,

k) at least some of the CCD-columns ($b_1$ to $b_m$) are driven individually or combined to form groups ($b_{2/1}$, $b_{4/1}$...$b_{10/1}$; $b_{3/2}$, $b_{7/2}$, $b_{11/2}$) by way of separate clock inputs with different clock frequencies ($t_1$, $t_2$, $t_3$...$t_k$).

2. An X-ray diagnostics device according to claim 1, characterised in that the different driving of the CCD-columns ($b_1$...$b_m$) is concentrated in certain surface regions of the CCD-sensor (11) or of the sensor arrangement (12,13).

3. An X-ray diagnostics device according to claim 1 or 2, characterised in that the clock frequencies ($t_1$ to $t_k$) are able to be divided with the aid of a clock generator (22) so that a curved layer is recorded from the jaw.

4. An X-ray diagnostics device according to one of claims 1 to 3, characterised in that through electric decoupling of charges on the edge of the image zone (11a) of the CCD-sensor (11) the width of the active sensor area (8) and therefore the depth of focus of the layer produced is able to be altered.

5. An X-ray diagnostics device according to claim 4, characterised in that the decoupling of the charges occurs by interrupting the charge transfer, for which purpose the CCD-sensor (11) contains integrated analog switches, which divert certain charges to the substrate of the CCD-sensor or to earth.

**Revendications**

1. Appareil de radiodiagnostic dentaire pour l'établissement de tomographies panoramiques de la mâchoire d'un patient, présentant les caractéristiques suivantes :

a) une unité (1) pouvant tourner autour d'un axe vertical et qui supporte, d'une part, une source de rayons X (3), et, d'autre part, un diaphragme (7) comportant une fente secondaire,

b) des moyens (9) pour convertir les rayons X en des rayons visibles,

c) un dispositif de détection (11) servant à former des signaux électriques proportionnellement à l'intensité du rayonnement,

d) un convertisseur analogique/numérique (16) raccordé au dispositif de détection et qui numérise les tensions délivrées par le dispositif de détection,

e) une mémoire d'images (18),

f) un dispositif de traitement des données comportant un calculateur (21), qui calcule une image formant vue d'ensemble à partir des signaux délivrés par le dispositif de détection pendant le déroulement d'un enregistrement,

g) un dispositif de reproduction d'images (20) raccordé au dispositif de traitement des données,

h) le dispositif de détection comporte un ou plusieurs capteurs CCD (11), qui sont disposés de telle sorte que l'image de la fente secondaire (8) peut être formée sur leur zone d'image (11a), l'image d'une ligne étant formée dans la direction d'étendue longitudinale de la fente sur une ligne CCD,

i) au capteur CCD (11) est raccordé un générateur de cadence (22), grâce aux impulsions de cadence duquel des images de charges (LB), qui correspondent à des images lumineuses (LiB) dans la fente secondaire (8), sont additionnées pour former une image de charges d'ensemble (Lbges.), les images de charges (LB) étant éventuellement transférées de la zone d'image (11a) dans une zone de mémoire (11b), puis lues par l'intermédiaire d'un registre à décalage (11c) et ensuite envoyées au transducteur analogique/numérique (16),

j) la fréquence de cadence ($f_{cadence}$) du générateur de cadence (22) est choisie de telle sorte que les images de charges (LB) sont délivrées de façon cadencée ligne par ligne par l'intermédiaire du registre à décalage (11c), avec la même vitesse (v) que celle avec laquelle un film radiographique est déplacé par rapport à la fente secondaire (8) dans une technique d'enregistrement classique,

k) au moins quelques-unes des colonnes CCD ($b_1$ à $b_m$) sont commandées individuellement ou en étant réunies selon des groupes ($b_{2/1}$, $b_{4/1}$ ... $b_{10/1}$; $b_{3/2}$, $b_{7/2}$, $b_{11/2}$), par l'intermédiaire d'entrées de cadence séparées, avec des cadences différentes ($t_1$, $t_2$, $t_3$ ... $t_k$).

2. Appareil de radiodiagnostic suivant la revendication 1, caractérisé par le fait que la commande différente des colonnes CCD ($b_1$ ... $b_m$) est concentrée sur des zones superficielles déterminées du capteur CCD (11) ou du dispositif de capteur (12,13).

3. Appareil de radiodiagnostic suivant la revendication 1 ou 2, caractérisé par le fait que les fréquences de cadence ($t_1$ à $t_k$) peuvent être divisées au moyen d'un générateur de cadence (22), en sorte qu'une couche cintrée de la mâchoire est enregistrée.

4. Appareil de radiodiagnostic suivant l'une des revendications 1 à 3, caractérisé par le fait que la largeur de la surface active (8) du capteur et, par Conséquent, la profondeur de champ de la couche de champ peuvent être modifiées grâce à un découplage électrique de charges au niveau du bord de la zone d'image (11a) du capteur CCD (11).

5. Appareil de radiodiagnostic suivant la revendication 4, caractérisé par le fait que le découplage des charges est obtenu au moyen d'une interruption du transport de charges, le capteur CCD (11) contenant, à cet effet, des interrupteurs analogiques intégrés, qui évacuent des charges déterminées en direction du substrat du capteur CCD ou en direction de la masse.

FIG 1

FIG 2

FIG 3

FIG 4

FIG 5

FIG 6

EP 0 357 944 B1

FIG 7

FIG 8

FIG 9

FIG 10